# EUROPEAN PATENT APPLICATION

(11) **EP 3 977 992 A1**
(43) Date of publication of application: **06.04.2022**
(21) Application number: 20814275.2
(22) Date of filing: 26.05.2020
(51) Int. Cl.: A61K 31/4174, A61K 9/06, A61K 9/70, A61K 47/10, A61K 47/12, A61K 47/14, A61P 25/20

(54) **EXTERNAL PREPARATION**

(30) Priority: 27.05.2019 JP 2019098548
(71) Applicant: Kyukyu Pharmaceutical Co., Ltd., Tokyo 103-0023 (JP); Maruishi Pharmaceutical Co., Ltd., Osaka 538-0042 (JP)
(72) Inventor: UCHITOMI, Ryo, Imizu-shi, Toyama 939-0351 (JP); YAMAZAKI, Yuhiro, Imizu-shi, Toyama 939-0351 (JP)
(74) Representative: Hartz, Nikolai
(86) International application number: PCT/JP2020/020642
(87) International publication number: WO 2020/241605

(57) **Abstract**

Provided is a dexmedetomidine-containing non-aqueous external preparation being capable of suppressing the precipitation of a crystal of dexmedetomidine in the preparation, and having satisfactory transdermal absorbability. The non-aqueous external preparation includes: (A) dexmedetomidine or a salt thereof; (B) an aliphatic alcohol having 10 to 12 carbon atoms; (C) a propylene glycol monoester of a fatty acid having 6 to 16 carbon atoms; (D) an organic acid; and (E) an organic acid salt.

## Description

### Field of the Invention

The present invention relates to a non-aqueous external preparation containing dexmedetomidine or a salt thereof, which is useful as a sedative.

### Background of the Invention

Dexmedetomidine or a salt thereof is an agonist of an α₂ adrenergic receptor, has a sedative action, an analgesic action, and a sympathoinhibitory action, and is used as a sedative. Currently, dexmedetomidine or the salt thereof is used for sedation during, and after withdrawal from, artificial ventilation in intensive care in Japan, and is widely used as a sedative or an analgesic in other countries. Only intravenous administration is approved as a dosage form of the dexmedetomidine.

Various transdermally absorbable preparations are being investigated as dosage forms of dexmedetomidine. For example, there are reports of: a non-aqueous patch including a backing layer, an anchor adhesive layer, a porous intermediate layer, a contact adhesive layer, and a release liner (Patent Literature 1); a reservoir-type patch (Patent Literature 2); a patch using a cyclodextrin derivative (Patent Literature 3); a patch having dexmedetomidine blended therein as a salt with a carboxylic acid (Patent Literature 4); a hydrous patch containing dexmedetomidine or a salt thereof and a water-soluble polymer (Patent Literature 5); and a transdermal delivery device containing dexmedetomidine and a pressure sensitive adhesive (Patent Literature 6).

[PTL 1] JP-B2-3043064
[PTL 2] JP-B2-3011459
[PTL 3] JP-B2-3734267
[PTL 4] JP-B2-3483881
[PTL 5] WO-A1-2015/093503
[PTL 6] JP-A-2016-532642

### Summary of the Invention

### Technical Problem

However, in each of related-art dexmedetomidine-containing external preparations, in order to achieve high transdermal absorbability, a complicated preparation configuration or release mechanism is used, or dexmedetomidine is used in a special salt form. In addition, Patent Literature 5 is directed to a hydrous patch, and hence has a limitation in terms of pressure-sensitive adhesive property. In addition, a non-aqueous external preparation had a problem in that a crystal of dexmedetomidine was precipitated in the preparation.

Accordingly, an object of the present invention is to provide a dexmedetomidine-containing non-aqueous external preparation being capable of suppressing the precipitation of a crystal of dexmedetomidine in the preparation, and having satisfactory transdermal absorbability.

### Solution to Problem

In view of the foregoing, the inventors of the present invention made various investigations in order to obtain a non-aqueous external preparation of dexmedetomidine, the preparation being capable of suppressing the precipitation of a crystal of dexmedetomidine, and having satisfactory transdermal absorbability, and as a result, found that the suppression of the precipitation of a crystal of dexmedetomidine and satisfactory transdermal absorbability can be achieved by blending the following four kinds of components into a non-aqueous base containing dexmedetomidine: an aliphatic alcohol having 10 to 12 carbon atoms; a propylene glycol monoester of a fatty acid having 6 to 16 carbon atoms; an organic acid; and an organic acid salt. Thus, the present invention was completed.

That is, the present invention provides the following items [1] to [3].
[1] A non-aqueous external preparation, comprising: (A) dexmedetomidine or a salt thereof; (B) an aliphatic alcohol having 10 to 12 carbon atoms; (C) a propylene glycol monoester of a fatty acid having 6 to 16 carbon atoms; (D) an organic acid; and (E) an organic acid salt.
[2] The external preparation according to Item [1], wherein the non-aqueous external preparation is an ointment or a non-aqueous patch.
[3] The external preparation according to Item [1] or [2], wherein the non-aqueous external preparation is a non-aqueous patch.

### Advantageous Effects of the Invention

The external preparation of the present invention is capable of suppressing the precipitation of a crystal of dexmedetomidine in the preparation, and has satisfactory transdermal absorbability. Accordingly, the effect of dexmedetomidine as a sedative or a drug for any of various indications (examples thereof include, but not limited to, ADHD, anxiety, insomnia, alcohol and other withdrawal syndromes, and pain control) based on a central α₂ adrenergic receptor-stimulating action can be stably obtained for a long period of time.

### Detailed Description of the Invention

A non-aqueous external preparation of the present invention comprises: (A) dexmedetomidine or a salt thereof; (B) an aliphatic alcohol having 10 to 12 carbon atoms; (C) a propylene glycol monoester of a fatty acid having 6 to 16 carbon atoms; (D) an organic acid; and (E) an organic acid salt.

Dexmedetomidine or the salt thereof serving as the component (A) is an active ingredient of the external preparation of the present invention. Dexmedetomidine has the chemical name (+)-(S)-4-[1-(2,3-dimethylphenyl)ethyl]-1H-imidazole, and is an agonist of a central α₂ adrenergic receptor. Examples of the salt of dexmedetomidine include acid addition salts. Of those, an inorganic acid addition salt with hydrochloric acid, sulfuric acid, nitric acid, phosphoric acid, or the like is preferred, and hydrochloride is more preferred.

The content of dexmedetomidine or the salt thereof is preferably from 0.1 mass% to 10 mass%, more preferably from 0.1 mass% to 7 mass%, even more preferably from 0.1 mass% to 5 mass% in the non-aqueous external preparation (in an adhesive gel base in the case of a patch), from the viewpoints of a crystal precipitation-suppressing effect, a transdermal absorption-enhancing effect, and a skin irritation-reducing effect.

The aliphatic alcohol having 10 to 12 carbon atoms serving as the component (B) and the propylene glycol monoester of a fatty acid having 6 to 16 carbon atoms serving as the component (C) particularly exhibit a crystal precipitation-suppressing effect on dexmedetomidine or the salt thereof by virtue of blending these two components in combination.

Examples of the aliphatic alcohol having 10 to 12 carbon atoms (B) include capric alcohol (decanol) and lauryl alcohol (dodecanol). Of those, lauryl alcohol is particularly preferred. Herein, the aliphatic alcohols each having 10 to 12 carbon atoms may be used alone or in combination thereof.

The content of the component (B) is preferably from 0.5 mass% to 10 mass%, more preferably from 1 mass% to 8 mass%, even more preferably from 1 mass% to 6 mass% in the non-aqueous external preparation (in an adhesive gel base in the case of a patch), from the viewpoints of a crystal precipitation-suppressing effect, a transdermal absorption-enhancing effect, and a skin irritation-reducing effect on dexmedetomidine or the salt thereof.

Examples of the propylene glycol monoester of a fatty acid having 6 to 16 carbon atoms serving as the component (C) include propylene glycol monocaproic acid ester, propylene glycol monocaprylic acid ester, propylene glycol monocapric acid ester, propylene glycol monolauric acid ester, propylene glycol monomyristic acid ester, and propylene glycol monopalmitic acid ester. Of those, a propylene glycol monoester of a fatty acid having 8 to 14 carbon atoms is more preferred, and a propylene glycol monoester of a fatty acid having 8 to 12 carbon atoms is even more preferred. Those propylene glycol monoesters of fatty acids each having 6 to 16 carbon atoms may be used alone or in combination thereof.

The content of the component (C) is preferably from 1 mass% to 12 mass%, more preferably from 1 mass% to 10 mass%, even more preferably from 1 mass% to 8 mass% in the non-aqueous external preparation (in an adhesive gel base in the case of a patch), from the viewpoints of a crystal precipitation-suppressing effect and a transdermal absorption-enhancing effect on dexmedetomidine or the salt thereof.

The organic acid serving as the component (D) and the organic acid salt serving as the component (E) improve the transdermal absorbability of dexmedetomidine or the salt thereof by virtue of blending these two components in combination. In addition, a crystal precipitation-suppressing effect on dexmedetomidine or the salt thereof is exhibited by combining the component (B) and the component (C) with the component (D) and the component (E).

Examples of the organic acid (D) include: C₂ to C₅ monocarboxylic acids, such as acetic acid, propionic acid, and butyric acid; dicarboxylic acids, such as adipic acid, maleic acid, and succinic acid; hydroxycarboxylic acids, such as lactic acid, tartaric acid, malic acid, and citric acid; ketocarboxylic acids, such as acetoacetic acid and levulinic acid; and higher fatty acids, such as caprylic acid, capric acid, caproic acid, lauric acid, myristic acid, palmitic acid, stearic acid, isostearic acid, oleic acid, linoleic acid, linolenic acid, and behenic acid.

Examples of the organic acid salt (E) include alkali metal salts of the above-mentioned organic acids. Those organic acids (D) may be used alone or in combination thereof, and those organic acid salts (E) may be used alone or in combination thereof. In addition, the organic acid salt (E) may be a salt of the same organic acid as the organic acid (D), or may be a salt of an organic acid different therefrom.

Of those organic acids each serving as the component (D), one kind or two or more kinds selected from a C₂ to C₅ monocarboxylic acid, a C₂ to C₈ dicarboxylic acid, a C₂ to C₈ hydroxycarboxylic acid, a C₄ to C₈ ketocarboxylic acid, and a C₆ to C₂₄ fatty acid are more preferred. In addition, one kind or two or more kinds selected from a C₂ to C₅ monocarboxylic acid salt, a C₂ to C₈ dicarboxylic acid salt, a C₂ to C₈ hydroxycarboxylic acid salt, and a C₄ to C₈ ketocarboxylic acid salt are more preferred as the organic acid salt (E).

The content of the component (D) is preferably from 0.1 mass% to 5 mass%, more preferably from 0.1 mass% to 3 mass%, even more preferably from 0.1 mass% to 2 mass% in the non-aqueous external preparation (in an adhesive gel base in the case of a patch), from the viewpoint of a transdermal absorption-enhancing effect on dexmedetomidine or the salt thereof.

The content of the component (E) is preferably from 0.05 mass% to 5 mass%, more preferably from 0.05 mass% to 3 mass%, even more preferably from 0.05 mass% to 2 mass% in the non-aqueous external preparation (in an adhesive gel base in the case of a patch), from the viewpoint of a transdermal absorption-enhancing effect on dexmedetomidine or the salt thereof.

The external preparation of the present invention is a non-aqueous external preparation, and specific examples thereof include a non-aqueous preparation for transdermal absorption, such as an ointment, and a non-aqueous patch. Components that may be blended into the non-aqueous external preparation of the present invention other than the components (A) to (E) vary depending on those dosage forms.

In the case of a preparation for skin application, such as an ointment, any of various ointment bases may be blended in addition to the components (A) to (E). Examples of those components include: mineral bases, such as vaseline, paraffin, Plastibase, and silicone; and animal and plant bases, such as a plant oil, lard, beef tallow, and a wax.

In the case of a non-aqueous patch, for example, a non-aqueous base, a tackifier, a plasticizer, a solubilizing agent, an absorption enhancer other than the above-mentioned compounds, a stabilizer, a UV absorber, a filler, a colorant, a flavoring agent, or a contrastimulant (e.g., cholesterol or urea) may be blended.

Herein, examples of the non-aqueous base include a rubber base and an acrylic acid-based base. Examples of the rubber base include a styrene-isoprene-styrene block copolymer (SIS), polyisoprene, polyisobutylene (PIB), a styrene-butadienestyrene block copolymer (SBS), a styrene-butadiene copolymer (SBR), a natural rubber, and a silicone-based rubber. Examples of the acrylic acid-based base include an acrylic acid-octyl acrylate copolymer, an acrylic acid ester-vinyl acetate copolymer, a 2-ethylhexyl acrylate-vinylpyrrolidone copolymer, a 2-ethylhexyl acrylate-2-ethylhexyl methacrylate-dodecyl methacrylate copolymer, a methyl acrylate-2-ethylhexyl acrylate copolymer, and a methacrylic acid-n-butyl acrylate copolymer. Of those, a rubber-based base is more preferred.

Examples of the tackifier include a petroleum-based resin (an aliphatic hydrocarbon resin or an alicyclic hydrocarbon resin), a terpene resin, a rosin-based resin, a terpene phenol resin, a coumarone resin, a xylene resin, and a styrene resin.

Examples of the plasticizer include liquid paraffin, light liquid paraffin, polybutene, isopropyl myristate, octyldodecyl myristate, isopropyl palmitate, butyl stearate, myristyl lactate, dioctyl adipate, diethyl sebacate, diisopropyl sebacate, dioctyl sebacate, diisopropyl adipate, dioctyl succinate, octyldodecanol, hexyldecanol, castor oil, and triethyl citrate.

Examples of the solubilizing agent include glycerin, propylene glycol, triethylene glycol, polyethylene glycol (molecular weight: 200 to 4,000), 1,3-butanediol, crotamiton, squalane, squalene, a propylene glycol fatty acid ester, a medium-chain fatty acid triglyceride, a glycerin fatty acid ester, propylene carbonate, triacetin, and lauromacrogol.

Examples of the absorption enhancer other than the components (A) to (E) include caprylic acid, capric acid, caproic acid, lauric acid, myristic acid, palmitic acid, stearic acid, oleic acid, linoleic acid, linolenic acid, methyl laurate, isopropyl myristate, myristyl myristate, octyldodecyl myristate, cetyl palmitate, cetyl lactate, isopropyl palmitate, sorbitan monooleate, glycerin monolaurate, glycerin monooleate, sorbitan monolaurate, polysorbate 20, polysorbate 80, polyethylene glycol monolaurate, polyethylene glycol monostearate, polyoxyethylene hydrogenated castor oil, and α-monoisostearyl glycerin ester.

Examples of the stabilizer include L-ascorbic acid, L-ascorbyl palmitate, L-ascorbyl stearate, L-ascorbic acid-2-glucoside, sodium L-ascorbate, calcium L-ascorbate, magnesium L-ascorbyl phosphate, isoascorbic acid, sodium isoascorbate, dibutylhydroxytoluene, butylhydroxyanisole, propyl gallate, octyl gallate, lauryl gallate, nordihydroguaiaretic acid, trihydroxybutyrophenone, tert-butylhydroquinone, 4-hydroxymethyl-2,6-di-tert-butylphenol, sulfites, such as sodium pyrosulfite, sodium hydrogen sulfite, sodium sulfite, and potassium pyrosulfite, and mercaptobenzimidazole.

In the case of an ointment, the ointment may be produced by blending the components (A) to (E) into any of the above-mentioned ointment bases, melting the mixture through heating as required, and mixing and kneading the whole to homogeneity.

In the case of a non-aqueous patch, the non-aqueous patch may be produced by, for example, any one of: a hot-melt method involving adding a solution of a drug to a hot melt of a base component, and casting the mixture; and a solvent method involving adding a solution of a drug to a solution of a base component in an organic solvent, and applying and drying the mixture. Of those, a solvent method is preferred from the viewpoint that a high heat load is not applied to the drug. Examples of the solvent to be used in the solvent method include toluene, xylene, hexane, ethyl acetate, tetrahydrofuran, and dichloromethane. From the viewpoint of the toxicity of the organic solvent, ethyl acetate is preferred (the Guideline for Residual Solvents in Pharmaceuticals: PMSB/ELD Notification No. 307) .

In the case of the solvent method, the non-aqueous patch may be produced by, for example, the following procedure.

Dexmedetomidine hydrochloride, the organic acid salt, and the organic acid are added to liquid paraffin, and the mixture is sufficiently stirred until dexmedetomidine or the salt thereof is dissolved or turned into an oily substance, to thereby prepare an active ingredient mixed liquid.

When the organic acid salt is a crystalline compound or the like, in order to shorten the preparation time of the active ingredient mixed liquid, it may be appropriate to add both or one of water and ethanol, or to perform ultrasonic treatment.

When both or one of water and ethanol is added, if the amount thereof in the active ingredient mixed liquid is large, the base component may aggregate at the time of the addition of the active ingredient mixed liquid to a base component solution to be described later.

In such case, the aggregation of the base component can be prevented by evaporating the water and ethanol in the active ingredient mixed liquid through heating, pressure reduction, inert gas blowing, or the like as required.

A rubber base, a tackifier, a plasticizer, a solubilizing agent, an absorption enhancer containing the components (B) and (C), a stabilizer, and other additives are added to and dissolved in the organic solvent to prepare the base component solution.

The active ingredient mixed liquid is added to the base component solution, and the mixture is homogeneously stirred to serve as an application solution.

The application solution is applied at a predetermined thickness onto a liner, and then dried by blowing of hot air or the like. A support is bonded to the resultant pressure-sensitive adhesive layer, and the resultant is cut into a predetermined size to provide the preparation.

In this case, examples of the support include: a plastic film of polyethylene terephthalate, polypropylene, polyethylene, or the like; a product obtained by subjecting the surface of the plastic film to corona discharge treatment or sand mat treatment; and a product obtained by laminating, on the plastic film, a nonwoven fabric formed of polyethylene terephthalate, polypropylene, polyethylene, or the like.

In addition, an example of the liner is a product obtained by subjecting a film formed of polyethylene terephthalate, polyethylene, or polypropylene to silicone treatment or fluorine treatment.

The non-aqueous external preparation of the present invention is stable without causing the precipitation of a crystal of dexmedetomidine or the salt thereof even when stored for a long period of time, and has satisfactory transdermal absorbability. Accordingly, when the non-aqueous external preparation of the present invention is used, the effect of dexmedetomidine as a sedative or a drug for any of various indications (examples thereof include, but not limited to, ADHD, anxiety, insomnia, alcohol and other withdrawal syndromes, and pain control) based on a central α₂ adrenergic receptor-stimulating action can be stably obtained for a long period of time.

### Examples

Next, the present invention is described below in more detail by way of Examples. However, the present invention is not limited to these Examples.

### (Production Method for Non-aqueous Patch)

Preparations of formulations (mass%) shown in Table 1 to Table 3 were obtained by the following procedure.

### (i) Preparation of Base Solution

Base components shown in the tables were added to ethyl acetate (appropriate amount), and were dissolved therein by stirring to prepare a base solution.

### (ii) Preparation of Active Ingredient Mixed Liquid

An organic acid, an organic acid salt, and dexmedetomidine hydrochloride were added to liquid paraffin, and were dissolved therein by stirring to prepare an active ingredient mixed liquid. For the dissolution, as required, an appropriate amount of water/ethanol was added, and ultrasonic treatment was performed. Further, after the organic acid, the organic acid salt, and dexmedetomidine hydrochloride had been dissolved, water/ethanol was evaporated as required.

In Comparative Example 1, the active ingredient mixed liquid was prepared by adding dexmedetomidine hydrochloride to liquid paraffin and subjecting the mixture to ultrasonic dispersion.

### (iii) Preparation of Application Solution

The other components shown in the tables were added to the base solution, and the mixture was stirred. Then, the active ingredient mixed liquid was added, and the whole was stirred to prepare an application solution.

### (iv) Application, Drying, Cutting, and Packaging

The application solution was applied onto a liner (silicone-treated PET film), and dried. A support was bonded to the resultant pressure-sensitive adhesive layer, and the resultant was cut into a predetermined size (10 cm²) to provide a preparation. An application amount was adjusted so that the amount of dexmedetomidine hydrochloride per sheet of the preparation was 1 mg (1 mg/10 cm²). The support used was obtained by laminating a nonwoven fabric on a PET film, and the support was bonded so that the pressure-sensitive adhesive layer was brought into contact with its nonwoven fabric surface.

Sheets of the resultant preparation were packaged in aluminum bags at one sheet per bag.

### (Skin Permeability Test)

An abdominal skin excised from a hairless mouse (male, 7 weeks old) was mounted on a Franz diffusion cell (effective permeation area: 0.92 cm², receiver volume: 2.5 mL). A predetermined preparation sample was applied to a stratum corneum side, and an isotonic phosphate buffer (pH=6.8) was applied to a dermis side. During the test, the isotonic phosphate buffer in the receiver was kept at a constant temperature of 32°C, 1.0 mL thereof was collected at predetermined times, and an equal volume of the isotonic phosphate buffer was immediately added. The dexmedetomidine hydrochloride in the collected sample was quantified by high performance liquid chromatography to determine the cumulative amount of dexmedetomidine permeated per unit area.

### (Checking Method for Crystal Precipitation)

The packaged preparation was stored at 25°C. After the storage, the presence or absence of a crystal of dexmedetomidine in the preparation on the 5th day and the 30th day was observed as follows: the liner of the preparation was released to expose the adhesive gel base surface, followed by observation from the adhesive gel base side with an optical microscope (light transmission, from 100 times to 800 times, DIGITAL MICROSCOPE KH-8700, Hirox Co., Ltd.). Evaluation was performed in accordance with the following scores.
A: A crystal is not found or is found in an extremely small quantity.
B: A crystal is found.

**Table 1**

| Example/Comparative Example | | | Example 1 | Example 2 | Example 3 | Example 4 | Example 5 | Example 6 | Comparative Example 1 |
|---|---|---|---|---|---|---|---|---|---|
| Base solution | SIS block copolymer | | 27 | 27 | 27 | 27 | 27 | 27 | 27 |
| | Tackifier | | 36 | 36 | 36 | 36 | 36 | 36 | 36 |
| | Dibutylhydroxytoluene | | 0.5 | | | | | | 0.5 |
| | Mercaptobenzimidazole | | | 0.1 | 0.1 | 0.1 | 0.1 | 0.1 | |
| Active ingredient mixed liquid | Dexmedetomidine hydrochloride | | 1 | 1 | 1 | 1 | 1 | 1 | 1 |
| | Sodium acetate (anhydrate) | | 0.63 | 0.63 | 0.63 | 0.63 | 0.63 | | |
| | Sodium lactate | | | | | | | 0.86 | |
| | DL-Malic acid | | 0.17 | 0.17 | | | | | |
| | Citric acid | | | | 0.24 | | | | |
| | Levulinic acid | | | | | 0.15 | | | |
| | Oleic acid | | | | | | 0.36 | 0.36 | |
| | Liquid paraffin | | 16.9 | 17.3 | 17.23 | 17.32 | 17.11 | 16.88 | 17.7 |
| Other components | Lauryl alcohol | | 2.8 | 2.8 | 2.8 | 2.8 | 2.8 | 2.8 | 2.8 |
| | Isopropyl myristate | | 10 | 10 | 10 | 10 | 10 | 10 | 10 |
| | Propylene glycol monocaprylic acid ester | | 5 | 5 | 5 | 5 | 5 | 5 | 5 |
| Evaluation | Presence or absence of crystal | 25°C×5 days | A | A | A | A | A | A | B |
| | | 25°C×30 days | A | A | A | A | A | A | B |
| | Skin permeability test | 0 hours | 0.00 | 0.00 | 0.00 | 0.00 | 0.00 | 0.00 | 0.00 |
| | | 2 hours | 7.76 | 9.99 | 8.78 | 8.34 | 6.34 | 4.55 | 3.56 |
| | | 4 hours | 36.60 | 36.02 | 31.98 | 33.30 | 26.56 | 18.19 | 11.91 |
| | | 6 hours | 61.72 | 59.83 | 55.38 | 57.29 | 47.89 | 35.61 | 18.35 |
| | | 8 hours | 78.10 | 78.89 | 74.76 | 75.18 | 65.23 | 52.62 | 21.74 |

**Table 2**

| | Example/Comparative Example | | Example 7 | Example 8 | Example 9 | Comparative Example 2 | Comparative Example 3 | Comparative Example 4 | Comparative Example 5 |
|---|---|---|---|---|---|---|---|---|---|
| Base solution | SIS block copolymer | | 18 | 18 | 18 | 18 | 18 | 18 | 18 |
| | Tackifier | | 36 | 36 | 36 | 36 | 36 | 36 | 36 |
| | Dibutylhydroxytoluene | | 0.5 | 0.5 | 0.5 | 0.5 | 0.5 | 0.5 | 0.5 |
| | Mercaptobenzimidazole | | | | | | | | |
| Active ingredient mixed liquid | Dexmedetomidine hydrochloride | | 1 | 1 | 1 | 1 | 1 | 1 | 1 |
| | Sodium acetate (anhydrate) | | 0.63 | 0.63 | 0.63 | 0.63 | 0.63 | 0.63 | 0.63 |
| | Sodium lactate | | | | | | | | |
| | DL-Malic acid | | 0.17 | 0.17 | 0.17 | | 0.17 | | |
| | Citric acid | | | | | | | | |
| | Levulinic acid | | | | | | | | |
| | Oleic acid | | | | | | | | |
| | Liquid paraffin | | 27.9 | 25.9 | 30.9 | 31.07 | 33.7 | 30.87 | 28.87 |
| Other components | Lauryl alcohol | | 2.8 | 2.8 | 2.8 | 2.8 | | | |
| | Isopropyl myristate | | 10 | 10 | | 10 | 10 | 10 | 10 |
| | Propylene glycol monocaprylic acid ester | | 3 | 5 | 10 | | | 3 | 5 |
| Evaluation | Presence or absence of crystal | 25°C×5 days | A | A | A | B | B | B | B |
| | | 25°C×30 days | A | A | A | B | B | B | B |

**Table 3**

| Example/Comparative Example | | | Comparative Example 6 | Comparative Example 7 | Comparative Example 8 | Comparative Example 9 | Comparative Example 10 | Comparative Example 11 |
|---|---|---|---|---|---|---|---|---|
| Base solution | SIS block copolymer | | 18 | 18 | 18 | 18 | 18 | 18 |
| | Tackifier | | 36 | 36 | 36 | 36 | 36 | 36 |
| | Dibutylhydroxytoluene | | 0.5 | 0.5 | 0.5 | 0.5 | 0.5 | 0.5 |
| | Mercaptobenzimidazole | | | | | | | |
| Active ingredient mixed liquid | Dexmedetomidine hydrochloride | | 1 | 1 | 1 | 1 | 1 | 1 |
| | Sodium acetate (anhydrate) | | 0.63 | 0.63 | 0.63 | 0.63 | 0.63 | 0.63 |
| | Sodium lactate | | | | | | | |
| | DL-Malic acid | | 0.17 | | | 0.17 | 0.17 | 0.17 |
| | Citric acid | | | | | | | |
| | Levulinic acid | | | | | | | |
| | Oleic acid | | | | | | | |
| | Liquid paraffin | | 33.7 | 36.07 | 31.07 | 25.9 | 25.9 | 25.9 |
| Other components | Lauryl alcohol | | | 2.8 | 2.8 | | | 2.8 |
| | Myristyl alcohol | | | | | 2.8 | 2.8 | |
| | Isopropyl myristate | | | | | 10 | 10 | 10 |
| | Propylene glycol monocaprylic acid ester | | 10 | 5 | 10 | 5 | | |
| | Propylene glycol dicaprylic acid ester | | | | | | 5 | 5 |
| Evaluation | Presence or absence of crystal | 25°Cx5 days | B | B | B | B | B | B |
| | | 25°C×30 days | B | B | B | B | B | B |

As shown in Table 1, in each of Examples 1 to 6, in which all of the components (B) to (E) were added, no crystal of dexmedetomidine was found in the preparation, and the transdermal absorbability of dexmedetomidine was satisfactory. Meanwhile, in Comparative Example 1, in which the components (D) and (E) were not added, dexmedetomidine was crystallized in the preparation, and besides, its transdermal absorbability was low.

In addition, the organic acid added in Examples 1 and 2 was DL-malic acid, and also when the acid was changed to different organic acids as in Examples 3, 4, and 5, similar effects were obtained. Similarly, the organic acid salt added in Examples 1 to 5 was sodium acetate (anhydrate), and also when the salt was changed to a different organic acid salt as in Example 6, similar effects were obtained.

As shown in Table 2 and Table 3, similarly, in each of Examples 7 to 9, in which all of the components (B) to (E) were added, no crystal of dexmedetomidine was found in the preparation, and a satisfactory preparation was obtained. Meanwhile, as shown in Comparative Examples 2 to 11, when any one or more of the components (B) to (E) were not added, a crystal of dexmedetomidine was found in the preparation.

In particular, when the lauryl alcohol of Example 7 was changed to myristyl alcohol (Comparative Example 9), the crystallization of dexmedetomidine was not able to be suppressed. Similarly, when DL-malic acid serving as the component (D) of Example 9 was not added (Comparative Example 8), the crystallization of dexmedetomidine was not able to be suppressed. When the propylene glycol monocaprylic acid ester of Example 8 was changed to propylene glycol dicaprylic acid ester (Comparative Example 11), the crystallization of dexmedetomidine was not able to be suppressed.

## Claims

1. A non-aqueous external preparation, comprising:
(A) dexmedetomidine or a salt thereof;
(B) an aliphatic alcohol having 10 to 12 carbon atoms;
(C) a propylene glycol monoester of a fatty acid having 6 to 16 carbon atoms;
(D) an organic acid; and
(E) an organic acid salt.

2. The external preparation according to claim 1, wherein the non-aqueous external preparation is an ointment or a non-aqueous patch.

3. The external preparation according to claim 1 or 2, wherein the non-aqueous external preparation is a non-aqueous patch.

4. The external preparation according to any one of claims 1 to 3, wherein the component (B) is one kind or two kinds selected from capric alcohol and lauryl alcohol.

5. The external preparation according to any one of claims 1 to 4, wherein the component (C) is one kind or two or more kinds selected from propylene glycol monoesters of fatty acids each having 8 to 14 carbon atoms.

6. The external preparation according to any one of claims 1 to 5, wherein the component (D) is one kind or two or more kinds selected from a C₂ to C₅ monocarboxylic acid, a C₂ to C₈ dicarboxylic acid, a C₂ to C₈ hydroxycarboxylic acid, a C₄ to C₈ ketocarboxylic acid, and a C₆ to C₂₄ fatty acid.

7. The external preparation according to any one of claims 1 to 6, wherein the component (E) is one kind or two or more kinds selected from a C₂ to C₅ monocarboxylic acid salt, a C₂ to C₈ dicarboxylic acid salt, a C₂ to C₈ hydroxycarboxylic acid salt, and a C₄ to C₈ ketocarboxylic acid salt.

8. The external preparation according to any one of claims 1 to 7, wherein a content of the component (A) is from 0.1 mass% to 10 mass%.

9. The external preparation according to any one of claims 1 to 8, wherein a content of the component (B) is from 0.5 mass% to 10 mass%.

10. The external preparation according to any one of claims 1 to 9, wherein a content of the component (C) is from 1 mass% to 12 mass%.

11. The external preparation according to any one of claims 1 to 10, wherein a content of the component (D) is from 0.1 mass% to 5 mass%.

12. The external preparation according to any one of claims 1 to 11, wherein a content of the component (E) is from 0.05 mass% to 5 mass%.
